(19) Europäisches Patentamt
European Patent Office
Office européen des brevets

(11) Veröffentlichungsnummer: **0 127 758**
**B1**

(12) # EUROPÄISCHE PATENTSCHRIFT

(45) Veröffentlichungstag der Patentschrift: **03.02.88**

(51) Int. Cl.⁴: **A 61 K 6/08**, C 08 F 283/02 // (C08F283/02, 220:12)

(21) Anmeldenummer: **84104637.8**

(22) Anmeldetag: **25.04.84**

(54) **Polymerisierbare Dentalmassen und hieraus hergestellte Dentalformkörper.**

(30) Priorität: **07.05.83 DE 3316851**

(43) Veröffentlichungstag der Anmeldung: **12.12.84 Patentblatt 84/50**

(45) Bekanntmachung des Hinweises auf die Patenterteilung: **03.02.88 Patentblatt 88/5**

(84) Benannte Vertragsstaaten: **AT CH DE FR GB IT LI SE**

(56) Entgegenhaltungen:
**EP-A-0 011 734**
**EP-A-0 014 515**
**EP-A-0 036 272**
**DE-A-2 403 211**
**US-A-3 073 795**
**US-A-3 629 187**
**US-A-4 141 144**

**CHEMICAL ABSTRACTS, Band 96, Nr. 2, 11. Januar 1982, Seiten 338,339, Nr. 11715v, Columbus, Ohio, USA; & JP - A - 81 110 606 (TOKUYAMA SODA CO., LTD.) 01.09.1981**

(73) Patentinhaber: **BAYER AG, Konzernverwaltung RP Patentabteilung, D-5090 Leverkusen 1 Bayerwerk (DE)**

(72) Erfinder: **Podszun, Wolfgang, Dr., Wolfskaul 4, D-5000 Köln 80 (DE)**
Erfinder: **Bley, Fritjof, Dipl.- Ing., Hohbuchweg 21, D-8991 Achberg/Liebenweiler (DE)**
Erfinder: **Walkowiak, Michael, Dr., Albertus-Magnus- Strasse 10, D-5090 Leverkusen 1 (DE)**

**EP 0 127 758 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft polymerisierbare Massen für Dentalzwecke, welche ein unvernetztes Polymer mit einer Glasübergangstemperatur $\leqq$ 0°C enthalten, sowie hieraus hergestellte vernetzte Dentalformkörper, in denen das unvernetzte Polymer als innerer, migrationsbeständiger Weichmacher vorliegt.

Bei den aus CA 96, 11715 v bekannten Dentalmaterialien werden bifunktionelle Monomere eingesetzt, die vernetzte Polymere ergeben. Sie sind in dem System unlöslich und können als Füllstoffe verwendet werden.

In der US-A 36 29 187 werden Dentalmaterialien beschrieben, die aus einem (Meth)-acrylsäureester, einem Füllstoff und einem Addukt von 2,2'-Propan-bis-(3-(4-phenoxy) 1,2-hydroxypropan-1-methacrylat) bestehen. Bei dem Addukt handelt es sich um ein bifunktionelles Monomer, das zu vernetzten, ebenfalls in dem System unlöslichen Polymeren führt.

Kunststoffzähne bestehen üblicherweise aus Polymethylmethacrylat (im folgenden mit PMMA abgekürzt). Die Zahnherstellung erfolgt dabei im allgemeinen auf chemoplastischem Wege nach einem Pulver-Flüssigkeitsverfahren, bei dem eine plastische Masse aus einem PMMA-Perlpolymerisat als Pulverkomponente und einer Mischung von Methylmethacrylat und Ethylendimethacrylat als Flüssigkeitskomponente durch radikalische Polymerisation unter Formgebung ausgehärtet wird.

PMMA-Zähne haben sich vor allem aufgrund ihrer physiologischen Verträglichkeit und guten kosmetischen Eigenschaften seit langem bewährt. Hinsichtlich wichtiger mechanischer Eigenschaften, wie Härte und Verschleißfestigkeit, sind jedoch Verbesserungen gegenüber PMMA wünschenswert. In der DE-AS 2 462 271 werden dentale Formkörper mit verbesserten mechanischen Eigenschaften beschrieben, die ein polymerisiertes, bifunktionelles Dimethacrylat wie Bis-GMA oder ein Urethandimethacrylat in Kombination mit ausschließlich mikrofeinem Siliziumdioxid als anorganischem Füllstoff enthalten. Kunststoffzähne gemäß DE-AS 2 462 271 zeigen jedoch den anwendungstechnischen Nachteil, daß sie sich beim Einsetzen in die Prothese weniger fest mit der Prothesenbasis verbinden als übliche PMMA-Zähne.

Aus der Veröffentlichung von Hirasawa in "Reports of the Institute for Medical and Dental Engineering", 1968, Seiten 55-61 ist bekannt, daß Härte und Abriebfestigkeit von PMMA durch die Verwendung von mikrofeinem Siliziumdioxid-Füllstoff signifikant gesteigert werden können. Derartige Materialien sind jedoch in der beschriebenen Form wegen schlechter Verarbeitungseigenschaften und besonders wegen zu großer Sprödigkeit für die Verwendung als Dentalwerkstoff nicht geeignet.

Es wurde Dentalmaterial, enthaltend polyfunktionelle (Meth)-acrylsäureester als Bindemittel, 5 bis 50 Gew.-% an mikrofeinem anorganischem Füllstoff sowie gegebenenfalls weitere Monomere, anorganischen und/oder organischen Füllstoff, gefunden, das 0,5 bis 25 Gew.-% eines unvernetzten aliphatischen Polyesters mit einem Molekulargewicht von $10^3$ bis $5 \cdot 10^5$ und einer Glasübergangstemperatur $\leqq$ 0°C enthält.

Insbesondere wurde gefunden, daß ein Dentalwerkstoff mit sehr guten Verarbeitungseigenschaften und verbesserten mechanischen Eigenschaften durch den Einsatz von

(A) 20 bis 50 Gew.-Teilen, bevorzugt 25 bis 55 Gew.-Teilen, bezogen auf 100 Gew.-Teile des Werkstoffs, eines polymerisierbaren Gemisches aus mono- und polyfunktionellen Methacrylsäureestern sowie gegebenenfalls Acrylsäureestern und weiteren Monomeren;

(B) 20 bis 80 Gew.-Teilen, bevorzugt 25 bis 50 Gew.-Teilen eines im wesentlichen unvernetzten, mit mikrofeinem anorganischem Füllstoff homogen gefüllten Polymerisats;

(C) 0,5 bis 25 Gew.-Teilen, bevorzugt 1 bis 10 Gew.-Teilen eines unvernetzten Polymers mit einer Glasübergangstemperatur Tg $\leqq$ 0° und einem Molekulargewicht $M_w$ von $10^3$ - $5.10^5$ sowie gegebenenfalls zusätzlich

(D) bis zu 40 Gew.-Teilen eines mit Haftvermittlern präparierten anorganischen Füllstoffes

erhalten wird.

Das Gemisch aus mono- und polyfunktionellen (Meth)acrylsäureestern (Komponente A), besteht zu 2 bis 50 Gew.-%, bevorzugt 5 bis 25 Gew.-%, aus polyfunktionellen (Meth)acrylsäureestern als Vernetzer.

Als monofunktionelle Methacrylsäureester sind in erster Linie Alkylmethacrylate mit 1 bis 12 C-Atomen im Alkoholteil wie z. B. Methylmethacrylat, Ethylmethacrylat, i-Butylmethacrylat, t-Butylmethacrylat, Cyclohexylmethacrylat, Furfurylmethacrylat, Tetrahydrofurfurylmethacrylat, Decylmethacrylat und Laurylmethacrylat geeignet. Gut geeignet sind insbesondere Mischungen verschiedener Methacrylsäureester, wobei ein Anteil von mehr als 50 Gew.-% Methylmethacrylat (bezogen auf die Summe der eingesetzten monofunktionellen (Meth)acrylsäureester) besonders vorteilhaft ist.

Unter polyfunktionellen Methacrylsäureestern werden vor allem Dimethacrylate und Trimethacrylate verstanden.

Als Dimethacrylate seien beispielhaft genannt:

Neopentylglykol-dimethacrylat, 1,12-Dodekandimethacrylat, ferner Derivate des Bisphenol-A, wie 2,2-Bis[4-(2-hydroxy-3-methacryloyloxypropyl)-phenyl]-propan (Bis-GMA), sowie Urethandimethacrylate, wie sie z. B. in US-A-3 425 988, 3 709 866 und 3 629 187 beschrieben sind.

Bevorzugte Dimethacrylate sind Ethylenglykoldimethacrylat, Diethylenglykoldimethacrylat, Triethylenglykoldimethacrylat, sowie Butandioldimethacrylat.

Als Trimethacrylate kommen z. B. Glycerintrimethacrylat, Trimethylolpropantrimethacrylat oder Pentaerythrittrimethacrylat in Frage.

Neben den Methacrylaten können bis zu 20 Gew.-% (bezogen auf die gesamte Komponente A)) weitere Vinyl- bzw. Vinyliden-Monomere, wie beispielsweise die den obengenannten Methacrylaten analogen

Acrylate, Styrol, α -Methylstyrol, Acrylnitril oder Vinylacetat eingesetzt werden.

Die Komponente B besteht bevorzugt aus im wesentlichen unvernetztem Perlpolymerisat mit einer mittleren Teilchengröße $d_{50}$ von 20 bis 150 µm, vorzugsweise 30 bis 80 µm, das mit 10 bis 70 Gew.-%, vorzugsweise 20 bis 50 Gew.-%, an mikrofeinem oberflächenbehandeltem anorganischem Füllstoff homogen gefüllt ist. Für die Herstellung des Perlpolymerisats kommen beliebige (Meth)acrylsäureester bzw. anteilig (bis zu 20 Gew.-%, bezogen auf Perlpolymerisat) weitere Monomere der obengenannten Art in Frage, wobei jedoch im wesentlichen nur eine olefinische Gruppe aufweisende Verbindungen eingesetzt werden, so daß das Perlpolymerisat im wesentlichen unvernetzt ist und von (Meth)acrylsäureestern zumindest angelöst werden kann. Die Viskosität des für die Herstellung des Perlpolymerisates verwendeten Monomer (gemische)s liegt vorzugsweise im Bereich von 0,001 bis 0,09 Pa.s (1-90 cP) bei der Dispergiertemperatur.

Vorzugsweise besteht der Polymerenanteil der Komponente B aus PMMA-Homopolymerisat oder einem Copolymerisat von Methylmethacrylat mit Methacrylsäure- oder Acrylsäureester mit 2 bis 12 C-Atomen im Alkoholteil. Besonders bevorzugt sind beispielsweise Copolymerisate aus 60 bis 90 Gew.-% Methylmethacrylat und 40 bis 10 Gew.-% i-Butylmethacrylat. Das Molekulargewicht ($M_w$) des Polymeren liegt vorzugsweise im Bereich von $10^5$ - $5.10^6$.

Als mikrofeiner anorganischer Füllstoff (Teilchengröße bevorzugt 10 bis 500 µm) eignet sich besonders Siliziumdioxid. Das Siliziumdioxid kann beispielsweise durch Fällung oder durch Flammhydrolyseverfahren hergestellt werden. Neben reinem Siliziumdioxid können auch Mischungen von Siliziumdioxid, beispielsweise mit Aluminiumoxid, Boroxid, Titandioxid oder Zirkonoxid eingesetzt werden, sofern der $SiO_2$ -Anteil im Gemisch überwiegt. Besonders gut geeignet ist flammhydrolytisch gewonnenes Siliziumdioxid mit einer mittleren Teilchengröße (Primärteilchengröße) von 10 bis 40 µm und einer BET-Oberfläche von 30 bis 300, vorzugsweise 40 bis 200 $m^2/g$.

Geeignete Oberflächenbehandlungsmittel sind in erster Linie die als Haftvermittler an sich bekannten Silanverbindungen, die beispielsweise in den US-PSen 3 066 113 und 3 539 533 beschrieben werden. Es können gesättigte Silanverbindungen, wie beispielsweise Hexamethyldisilazan oder γ -Glycidoxypropyltrimethoxysilan angewendet werden; bevorzugt werden jedoch ungesättigte, polymerisierbare Silanverbindungen mit Vinyl- oder Vinylidengruppen eingesetzt, wie Vinyltriethoxysilan, Vinyltrimethoxysilan, γ-Methacryloxypropyltrimethoxysilan, γ -Methacryloxypropyl-tris-(2-methoxyethoxy)-silan und Vinyltriacetoxysilan.

Die Silanverbindung soll in Anteilen von 1 bis 25, vorzugsweise von 5 bis 20 Gew.-%, bezogen auf den mikrofeinen Füllstoff, angewendet werden. Die Silanisierungsreaktion kann in einem inerten Lösungsmittel, beispielsweise in Methylenchlorid oder Toluol, durchgeführt werden. In manchen Fällen, z. B. bei der Nachbehandlung mit Hexamethyldisilazan, ist es auch möglich, auf ein Lösungsmittel zu verzichten. Es ist jedoch besonders vorteilhaft, die Silanisierungsreaktion in den bei der Suspensionspolymerisation eingesetzten Monomeren durchzuführen und die Polymerisation direkt mit dem so erhaltenen Monomer-Füllstoffgemisch durchzuführen, ohne daß der silanisierte anorganische Füllstoff isoliert wird.

Die Komponente B ist über eine Suspensionspolymerisation, z. B. unter Verwendung eines Copolymerisates aus Methylmethacrylat und Methacrylsäure als Dispergator nach einem hier nicht beanspruchten Verfahren zugänglich. Die für die Suspensionspolymerisation eingesetzte Mischung aus Füllstoff und-Monomer(en) kann in üblichen Rührapparaturen, vorzugsweise unter Anwendung hoher Scherkräfte (z. B. Rührenergien von 1 bis 10 Watt/l), hergestellt werden. Die Mischung wird zweckmäßigerweise vor der Polymerisation einer Vakuumbehandlung von 1,33 Pa bis 40 kPa (0,01 bis 300 Torr), vorzugsweise 133 bis 13332 Pa (1 bis 100 Torr), unterzogen (vorzugsweise mindestens 2 Minuten). Die Vakuumbehandlung erfolgt bevorzugt bei Raumtemperatur, doch können auch höhere oder niedrigere Temperaturen angewendet werden.

Zur Aktivierung können übliche monomerlösliche Radikalbildner verwendet werden. Das aktivierte Füllstoff/Monomer-Gemisch wird vorteilhafterweise der in einem Reaktionsgefäß vorgelegten wäßrigen Dispergatorlösung langsam unter Rühren zugesetzt, wobei das Verhältnis Monomer- zu Wasserphase in der Regel 1:1 bis 1:10, bevorzugt 1:2 bis 1:5, beträgt. Danach wird die Polymerisation durch Erhitzen auf die Zerfallstemperatur des Initiators eingeleitet.

Die für die erfindungsgemäßen Dentalmassen und -körper charakteristischen unvernetzten Polymere mit einer Glasübergangstemperatur Tg = 0°C sind z. B. Produkte auf Basis von Polyurethanen, Polycarbonaten, Polyestern und/ oder Polyethern.

Gut geeignet sind beispielsweise aliphatische Polyester auf Basis von $C_4$-$C_{10}$-Dicarbonsäuren und $C_2$-$C_{10}$-Diolen, wie Polyester aus Adipinsäure und/oder Azelainsäure mit 1,2-Propandiol, 1,3-Butandiol, 1,4-Butandiol oder 1,6-Hexandiol und einem Molekulargewicht über 1000. Die Polyester können als Endgruppen freie OH-Gruppen enthalten oder acyliert sein.

Besonders bevorzugt sind aliphatische Polyester- und/ oder Polyethercarbonate, welche wiederkehrende Struktureinheiten der nachstehenden allgemeinen Formel (I) aufweisen:

$$\left[ \left( X-O-\underset{\underset{O}{\|}}{C}-O \right)_l \left( X^1-O-\underset{\underset{O}{\|}}{C}-O \right)_n \right]_m \qquad (I),$$

wobei

$X^1$ gleiche oder verschiedene aliphatische Polyesterreste mit einem Molekulargewicht von 200 bis 6000, vorzugsweise von 750 bis 3500, besonders bevorzugt von 1000 bis 2500, bedeutet,

X die Bedeutung von $X^1$ hat oder für gleiche oder verschiedene aliphatische Polyetherreste mit einem Molekulargewicht von 200 bis 20 000, vorzugsweise von 700 bis 10 000, besonders bevorzugt von 1000 bis 3000, steht,

$n = o$ oder eine ganze Zahl von 1 bis 20 ist,

l eine ganze Zahl von 1 bis 20 darstellt und

m eine ganze Zahl, vorzugsweise $\geqslant 20$, bedeutet,

wobei die Grenzviskosität $[\eta]$ in Tetrahydrofuran vorzugsweise 0,5 bis 2,5 $\frac{dl}{g}$, besonders bevorzugt 0,8 bis 1,5 dl/g ist.

Als mehrwertige aliphatische Alkohole für die dem Rest $X^1$ zugrundeliegenden Polyester kommen vorzugsweise, gegebenenfalls im Gemisch miteinander, z. B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol; 1,4-Bis-(hydroxy-methylcyclohexan), 2-Methyl-1,3-propandiol, Di-, Tri-, Tetra- und Polyethylenglykol, Di-, Tri-, Tetra- und Polypropylenglykol und Dibutylenglykol in Frage. Bevorzugt sind Gemische aus zweien dieser Alkohole, wobei besonders bevorzugt einer der Alkohole verzweigte Struktur aufweist. Beispiele hierfür sind Ethylenglykol/Butandiol oder Hexandiol/Neopentylglykol.

Als mehrwertige aliphatische Carbonsäuren für die dem Rest $X^1$ zugrundeliegenden Polyester kommen bevorzugt zweiwertige aliphatische Carbonsäuren wie z. B. Kohlensäure, Oxalsäure, Malonsäure, Bernsteinsäure, Adipinsäure, Korksäure, Azelainsäure, Sebazinsäure, Hexahydrophthalsäure, Glutarsäure oder deren Gemische in Frage. Anstelle der freien Carbonsäuren können auch deren Anhydride oder Ester mit niederen Alkoholen eingesetzt werden. Bevorzugt sind Adipinsäure-Polyester.

Als Lactone für die dem Rest $X^1$ zugrundeliegenden Polyester kommen z. B. γ-Butyrolacton, ν-Valerolacton, ε-Caprolacton, 7-Hydroxyhexansäure-lacton, oder 8-Hydroxyoctansäurelacton in Frage, die in an sich bekannter Weise zu Polyestern polyaddiert werden können.

Als Hydroxycarbonsäuren für die dem Rest $X^1$ zugrundeliegenden Polyester kommen z. B. β-Hydroxypropionsäure, γ-Hydroxybuttersäure, δ-Hydroxyvaleriansäure, ε-Hydroxycapronsäure, 7-Hydroxyhexansäure oder 4-Hydroxy-cyclohexancarbonsäure in Frage, die in an sich bekannter Weise zu Polyestern kondensiert werden können.

Als Polyetherreste X eignen sich vorzugsweise solche der allgemeinen Formel (II)

$$-(\underset{R^1}{\underset{|}{CH}})_a \left[ O-(\underset{R^1}{\underset{|}{CH}})_a \right]_b \qquad (II)$$

in der die Reste

$R^1$ jeweils gleich oder verschieden sind und unabhängig voneinander H oder $C_1$-$C_4$-Alkylreste, vorzugsweise H oder $CH_3$, bedeuten,

a eine ganze Zahl von 2 bis 10, vorzugsweise 2 oder 4, darstellt und

b eine ganze Zahl von 2 bis 350, insbesondere von 3 bis 250, bedeutet.

Beispiele dafür sind Poly-(ethylenoxid)-glykole, Poly-(1,2-propylenoxid)-glykole, Poly-(1,3-propylenoxid)-glykole, Poly-(1,2-butylenoxid)-glykole, Poly-(tetrahydrofuran)-glykole, die entsprechenden Poly-(pentylenoxid)-glykole, Poly-(hexamethylenoxid)-glykole, Poly(heptamethylenoxid)-glykole, Poly-(octamethylenoxid)glykole, Poly-(nonamethylenoxid)-glykole und die Copolymeren oder Blockmischpolymeren aus beispielsweise Ethylenoxid und Propylenoxid. Bevorzugte Polyetherreste X sind solche auf Basis von Ethylenoxid und/oder Propylenoxid.

Die Carbonatgruppen enthaltenden Verbindungen der Strukturformel (I) werden hergestellt durch Umsetzung der beschriebenen OH-Endgruppenhaltigen Polyester und Polyether mit Kohlensäure-bis-arylestern der Formel (III)

$$Ar-O-\underset{\underset{O}{\|}}{C}-O-Ar \qquad (III)$$

worin

Ar ein substituierter oder unsubstituierter Arylrest mit 6 bis 18 C-Atomen ist, wobei als Substituenten

insbesondere $C_1$-$C_4$-Alkyle, sowie Nitro- oder Halogengruppen in Frage kommen, oder mit Bis -arylcarbonaten der Formel (IV)

$$Ar-O-\underset{\underset{O}{\|}}{C}-O-Y-O-\underset{\underset{O}{\|}}{C}-O-Ar \qquad (IV)$$

worin

Y die Bedeutung von X und $X^1$ in Formel (I) oder von einem Carbonatgruppen enthaltenden Polyester oder Polyether mit der wiederkehrenden Struktureinheit (I) hat.

Die Umsetzung wird normalerweise bei Temperaturen von 110 bis 200°C in Gegenwart von Umesterungskatalysatoren wie beispielsweise Alkali- oder Erdalkaliphenolaten, Alkali- oder Erdalkalialkoholaten, tertiären Aminen wie beispielsweise Triethylendiamin, Morpholin, Pyrrolidon, Pyridin, Triethylamin oder Metallverbindungen wie Antimontrioxid, Zinkchlorid, Titantetrachlorid und Titansäuretetrabutylester durchgeführt, wobei der Katalysator vorzugsweise in Mengen zwischen 20 ppm und 200 ppm, bezogen auf das Gesamtgewicht der Reaktionskomponenten, eingesetzt wird.

Derartige Umsetzungsprodukte sind bekannt und beispielsweise in DOS 2 732 718 oder in DOS 2 712 435 und DOS 2 651 639 beschrieben.

Die Grenzviskosität [η] wird in Tetrahydrofuran bei 25°C gemessen und ist in dl/g angegeben (Definition siehe z. B. H.G. Elias "Makromoleküle", Hüthig & Wepf-Verlag, Basel, Seite 265).

Die Komponente D ist ein mit Haftvermittlern präparierter feinteiliger anorganischer Füllstoff mit einer mittleren Teilchengröße im Bereich von 0,01 bis 10 µm. Geeignet sind beispielsweise diejenigen anorganischen Füllstoffe, die bereits weiter oben als Bestandteile der Komponente B beschrieben wurden. Darüber hinaus können auch agglomerierte Kieselsäuren mit mittleren Teilchengrößen von z. B. 0,5 bis 10 µm oder gemahlene Gläser oder Quarz mit mittleren Teilchengrößen im Bereich von z. B. 1 bis 5 µm eingesetzt werden. Als Haftvermittler eignen sich die weiter oben beschriebenen Silanverbindungen; besonders bevorzugt ist γ-Methacryloxypropyltrimethoxysilan. Die eingesetzte Silanmenge beträgt bevorzugt 2 bis 25 Gew.-% (bezogen auf Komponente D), wobei die niedrigen Werte für die gröberen Füllstoffe und die höheren für besonders feinteilige Füllstoffe anzuwenden sind. Die Silanisierungsreaktion kann mit Vorteil in einem inerten Lösungsmittel wie Aceton oder Methylenchlorid durchgeführt werden. Für die Isolierung des präparierten Füllstoffs ist ein Sprühtrocknungsprozeß besonders gut geeignet.

Die erfindungsgemäßen Dentalwerkstoffe und -formkörper können neben den schon erwähnten Komponenten noch weitere, in Dentalmaterialien üblicherweise verwendete Bestandteile enthalten. So können beispielsweise zusätzlich an sich bekannte ungefüllte Perlpolymerisate, vorzugsweise in Mengen bis zu 15 Gew.-% (bezogen auf die Dentalmasse), eingesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die erfindungsgemäßen Dentalformkörper werden durch radikalische Polymerisation der Dentalmassen unter Formgebung hergestellt. Die Verarbeitung ist sowohl nach Injektionsverfahren als auch Prägeverfahren möglich und erfolgt im allgemeinen nach den üblichen Herstellungsmethoden für PMMA-Zähne, z. B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersäuredinitril. Gut geeignet sind auch Mischungen von Polymerisationsinitiatoren mit unterschiedlichen Zerfallstemperaturen.

Die erfindungsgemäßen Dentalformkörper sind auf Hochglanz polierbar. Sie zeigen gegenüber solchen auf Basis von PMMA deutlich verbesserte mechanische Eigenschaften, insbesondere eine höhere Härte und höhere Verschleißfestigkeit sowie einen erhöhten E-Modul. Aus dem erfindungsgemäßen Werkstoff hergestellte Zähne lassen sich in herkömmlicher Weise in Prothesen einsetzen, wobei eine wirksame und dauerhafte Verbindung von Zahn und Prothesenbasis erreicht wird.

**Beispiel**

Herstellung eines unvernetzten, mit mikrofeinem anorganischem Füllstoff homogen gefüllten Perlpolymerisates

1.1 Silanisierungslösung

480 g γ -Methacryloxypropyltrimethoxysilan, 968 g entionisiertes Wasser und 144 g Methacrylsäure werden 30 Minuten bei Raumtemperatur vermischt, wobei eine homogene Lösung gebildet wird.

1.2 Gemisch aus mikrofeinem Füllstoff und Monomer

In einem 8 l Rührreaktor werden die Silanisierungslösung 1.1 sowie 3240 g Methylmethacrylat und 360 g Isobutylmethacrylat vorgelegt. Unter Rühren mit hoher Drehzahl (ca. 400 UpM) werden 2400 g mikrofeines Siliziumdioxid (mittlere Teilchengröße 30 nm, BET-Oberfläche = 130 $m^2/g$) portionsweise zugesetzt. Das Gemisch wird 12 Stunden bei Raumtemperatur weiter gerührt, wobei die Viskosität der Mischung laufend

erniedrigt wird. Anschließend wird das Gemisch 5 Minuten lang mit einem Vakuum von 15 Torr behandelt und mit Stickstoff belüftet.

Das Gemisch 1.2 wird unmittelbar vor der Weiterverarbeitung mit 24 g Benzoylperoxid versetzt und durch Erhöhen des $N_2$-Druckes in den vorbereiteten 40 l Reaktionsautoklaven gedrückt.

1.3 Perlpolymerisat

In einem 40 l Autoklaven mit Ankerimpeller-Rührer werden unter Stickstoffspülung 12,6 kg dest. Wasser und 5,4 kg Dispergatorlösung (8,5 % wäßrige, NaOH-alkalische Lösung eines Copolymerisates aus gleichen Gewichtsteilen Methylmethacrylat und Methacrylsäure) vorgelegt und 5 Minuten gemischt. Bei laufendem Rührer (250 UpM) wird das oben beschriebene Monomergemisch innerhalb von 15 Minuten eingedrückt. Nach beendeter Zugabe wird 2 Stunden bei Raumtemperatur gerührt. Anschließend wird 5 bar $N_2$ aufgedrückt und die Temperatur innerhalb von 15 Minuten auf 80° C (innen) erhöht. Bei einsetzender exothermer Reaktion (ca. 30 Minuten nach Aufheizbeginn) wird so stark gekühlt, daß die Innentemperatur im Bereich von 80 bis 90° C gehalten wird. Nach dem Abklingen der Reaktion wird 3 Stunden den bei 80° C nachgerührt. Das feste Perlpolymerisat wird nach dem Abkühlen durch Abdekantieren von feinteiligen Anteilen befreit, anschließend filtriert, mehrfach mit destilliertem Wasser gewaschen und bei 80° C getrocknet. Man erhält 6000 g transparente Perlen mit einem mittleren Teilchendurchmesser von 55 µm und einem $SiO_2$ -Gehalt von 38 %.

**Beispiel 2**

Erfindungsgemäßer Dentalwerkstoff

In einem Gemisch aus 80 Gew.-Teilen Methylmethacrylat und 20 Gew.-Teilen Ethylenglykoldimethacrylat werden 10 Gew.-Teile eines Polycarbonatgruppen enthaltenden Polyesters (Beispiel 3 der DE-OS 2 732 718) und 0,5 Gew.-Teile Dibenzoylperoxid gelöst.

In diese Lösung werden 100 Gew.-Teile Perlpolymerisat aus Beispiel 1 und 60 Gew.-Teile mikrofeines, silanisiertes Siliziumdioxid (mittlere Primärteilchengröße = 30 nm, BET-Oberfläche = 130 $m^2/g$, silanisiert mit 20 % Methacryloxypropyltrimethoxysilan) eingerührt, wobei eine zähe, leicht klebrige Masse ensteht. Das Gemisch erhält durch 2-stündige Temperung bei 40° C eine plastische, teigartige Konsistenz.

Das Gemisch wird in Formen gespritzt bzw. geprägt und bei 130° C in 6 Minuten ausgehärtet.

**Patentansprüche**

1. Dentalmaterial, enthaltend polyfunktionelle (Meth) acrylsäureester als Bindemittel, 5 bis 50 Gew.-% an mikrofeinem anorganischem Füllstoff sowie gegebenenfalls weitere Monomere, anorganischen und/oder organischen Füllstoff, dadurch gekennzeichnet, daß es 0,5 bis 25 Gew.-% eines unvernetzten aliphatischen Polyesters mit einem Molekulargewicht von $10^3$ bis $5 \cdot 10^5$ und einer Glasübergangstemperatur $\leq$ 0° C enthält.

2. Dentalmaterial nach Anspruch 1, dadurch gekennzeichnet, daß es

(A) 20 bis 50 Gew.-% eines Gemisches aus mono- und polyfunktionellen Methacrylsäureestern sowie gegebenenfalls weiteren Monomeren,

(B) 20 bis 60 Gew.-% eines im wesentlichen unvernetzten, mit mikrofeinem anorganischem Füllstoff homogen gefüllten Polymerisats auf Basis von (Meth)acrylsäureestern und gegebenenfalls weiteren Monomeren,

(C) 0,5 bis 25 Gew.-% eines unvernetzten Polyesters mit einer Glasübergangstemperatur $\leq$ 0° C und einem Molekulargewicht $M_w$ von $10^3 - 5 \cdot 10^5$, sowie gegebenenfalls

(D) 0 bis 40 Gew.-% eines mit Haftvermittlern behandeltes feinteiligen anorganischen Füllstoffs enthält.

3. Dentalmaterial nach Anspruch 1 oder 2, dadurch gekennzeichnet, daß der mikrofeine anorganische Füllstoff eine Teilchengröße von 10 bis 500 nm und eine BET-Oberfläche von 30 bis 300 $m^2/g$ aufweist.

4. Dentalmaterial nach Ansprüchen 1 bis 3, dadurch gekennzeichnet, daß der mikrofeine anorganische Füllstoff überwiegend $SiO_2$ mit einer mittleren Teilchengröße von 10 bis 40 nm und einer BET-Oberfläche von 40 bis 200 $m^2/g$ ist.

5. Dentalmaterial nach Ansprüchen 1 bis 4, dadurch gekennzeichnet, daß der mikrofeine anorganische Füllstoff silanisiert ist.

6. Dentalmaterial nach Ansprüchen 1 bis 5, dadurch gekennzeichnet, daß der unvernetzte Polyester ein Polyester(ether)carbonat mit wiederkehrenden Struktureinheiten der allgemeinen Formel

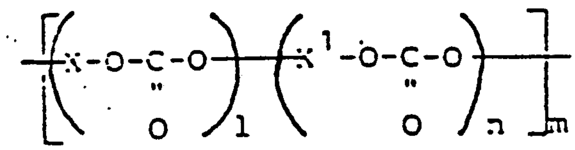

ist, in der

$X^1$ gleiche oder verschiedene aliphatische Polyesterreste mit einem Molekulargewicht von 200 bis 6000, vorzugsweise von 750 bis 3500, bedeutet,

X die Bedeutung von $X^1$ hat oder für gleiche oder verschiedene aliphatische Polyetherreste mit einem Molekulargewicht von 200 bis 20 000, vorzugsweise von 700 bis 10 000, steht,

n = 0 oder eine ganze Zahl von 1 bis 20, vorzugsweise 10 bis 20, ist,

l eine ganze Zahl von 1 bis 20, vorzugsweise von 10 bis 20, darstellt und

m eine ganze Zahl, vorzugsweise = 20, bedeutet,

wobei die Grenzviskosität des Polymers in Tetrahydrofuran vorzugsweise 0,5 bis 2,5 dl/g beträgt.

7. Dentalmaterial nach Ansprüchen 2 bis 6, dadurch gekennzeichnet, daß Komponente (A) zu 50 bis 98 Gew.-% aus monofunktionellen (Meth)acrylsäureestern und zu 2 bis 50 Gew.-% aus polyfunktionellen (Meth)acrylsäureestern als Vernetzer besteht.

8. Dentalmaterial nach Ansprüchen 2 bis 7, dadurch gekennzeichnet, daß Komponente (B) ein Perlpolymerisat mit einer mittleren Teilchengröße von 5 bis 150 µm ist.

9. Dentalformkörper, erhältlich durch radikalische Polymerisation von Dentalmaterialien gemäß Ansprüchen 1 bis 8 unter Formgebung.

10. Verfahren zur Herstellung von Dentalmaterial, enthaltend polyfunktionelle (Meth)acrylsäureester als Bindemittel, 5 bis 50 Gew.-% an mikrofeinem anorganischem Füllstoff sowie gegebenenfalls weitere Monomere, anorganischen und/oder organischen Füllstoff, dadurch gekennzeichnet, daß man einen unvernetzten aliphatischen Polyester mit einem Molekulargewicht von $10^3$ bis $5 \cdot 10^5$ und einer Glasübergangstemperatur von $\leqq 0°C$ einsetzt und polymerisiert.

**Claims**

1. Dental material, containing polyfunctional (meth)acrylic esters as binders 5 to 50 % by weight of microfine inorganic filler and, where appropriate, other monomers and an inorganic and/or organic filler, characterized in that it contains 0.5 to 25 % by weight of a non-crosslinked aliphatic polyester having a molecular weight of $10^3$ - $5x10^5$ and a glass transition temperature $\leqslant 0°C$.

2. Dental material according to Claim 1, characterized in that it contains

(A) 20 to 50 % by weight of a mixture of monofunctional and polyfunctional methacrylic esters and, where appropriate, other monomers,

(B) 20 to 60 % by weight of an essentially non-crosslinked polymer homogeneously filled with microfine inorganic filler and based on (meth)acrylic esters and, where appropriate, other monomers,

(C) 0.5 to 25 % by weight of a non-crosslinked polyester having a glass transition temperature $\leqslant 0°C$ and a molecular weight $M_w$ of $10^3$ - $5x10^5$ and, where appropriate,

(D) 0 to 40 % by weight of a finely divided inorganic filler treated with adhesion promoters.

3. Dental material according to Claim 1 or 2, characterized in that the microfine inorganic filler has a particle size of 10 to 500 nm and a BET surface area of 30 to 300 $m^2/g$.

4) Dental material according to Claims 1 to 3, characterized in that the microfine inorganic filler is predominantly $SiO_2$ having a mean particle size of 10 to 40 nm and a BET surface area of 40 to 200 $m^2/g$.

5. Dental material according to Claims 1 to 4, characterized in that the microfine inorganic filler is silanized.

6. Dental material according to Claims 1 to 5, characterized in that the non-crosslinked polyester is a polyester(ether) carbonate having repeating structural units of the general formula

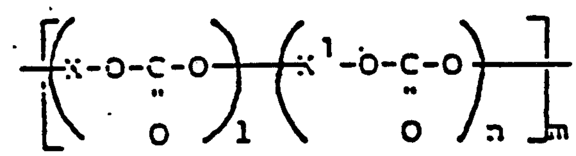

in which

$X^1$ denotes identical or different aliphatic polyester moieties having a molecular weight from 200 to 6,000, preferably from 750 to 3,500,

X has the meaning of $X^1$ or represents identical or different aliphatic polyether moieties having a molecular

weight from 200 to 20,000, preferably from 700 to 10,000,
n is 0 or an integer from 1 to 20, preferably 10 to 20,
l represents an integer from 1 to 20, preferably from 10 to 20, and
m denotes an integer, preferably 20,
the intrinsic viscosity of the polymer in tetrahydrofuran preferably being 0.5 to 2.5 dl/g.

7. Dental material according to Claims 2 to 6, characterized in that component (A) consists, to the extent of 50 to 98 % by weight, of monofunctional (meth)acrylic esters and, to the extent of 2 to 50 % by weight, of polyfunctional (meth)acrylic esters as crosslinkers.

8. Dental material according to Claims 2 to 7, characterized in that component (B) is a bead polymer having a mean particle size of 5 to 150 µm.

9. Moulded dental article obtainable by free radical polymerization of dental materials according to Claims 1 to 8, while moulding.

10. Process for the production of dental material, containing polyfuncitonal (meth)acrylic esters as binders, 5 to 50 % by weight of microfine inorganic filler and, where appropriate, other monomers and an inorganic and/or organic filler, characterized in that a non-crosslinked aliphatic polyester having a molecular weight of $10^3$ - $5x10^5$ and a glass transition temperature of $\leqslant 0°C$ is used and is polymerized.

## Revendications

1. Matériau dentaire contenant en tant que liant des esters (méth)acryliques polyfonctionnels, de 5 à 50 % en poids d'une matière de charge minérale microfine et le cas échéant d'autres monomères, une matière de charge minérale et/ou organique, caractérisé en ce qu'il contient de 0,5 à 25 % en poids d'un polyester aliphatique non réticulé de poids moléculaire $10^3$ à $5 \cdot 10^5$ présentant une température de transition du second ordre inférieure ou égale à 0° C.

2. Matériau dentaire selon la revendication 1, caractérisé en ce qu'il contient
(A) 20 à 50 % en poids d'un mélange d'esters méthacryliques mono- et polyfonctionnels et le cas échéant d'autres monomères,
(B) 20 à 60 % en poids d'un polymère essentiellement non réticulé, chargé de manière homogène par une matière de charge minérale microfine, à base d'esters (méth)acryliques et le cas échéant d'autres monomères,
(C) 0,5 à 25 % en poids d'un polyester non réticulé ayant une température de transition du second ordre inférieure ou égale à 0° C et un poids moléculaire $M_w$ de $10^3$ à $5 \cdot 10^5$, et le cas échéant
(D) 0 à 40 % en poids d'une matière de charge minérale en fines particules traitée par des agents d'adhérence.

3. Matériau dentaire selon la revendication 1 ou 2, caractérisé en ce que la matière de charge minérale microfine a une dimension de particule de 10 à 500 nm et une surface BET de 30 à 300 $m^2/g$.

4. Matériau dentaire selon les revendications 1 à 3, caractérisé en ce que la matière de charge minérale microfine consiste principalement en $SiO_2$ à une dimension de particule moyenne de 10 à 40 nm et une surface BET de 40 à 200 $m^2/g$.

5. Matériau dentaire selon les revendications 1 à 4, caractérisé en ce que la matière de charge minérale microfine est silanisée.

6. Matériau dentaire selon les revendications 1 à 5, caractérisé en ce que le polyester non réticulé est un polyester (éther)carbonate ayant des motifs de structure répétés de formule générale

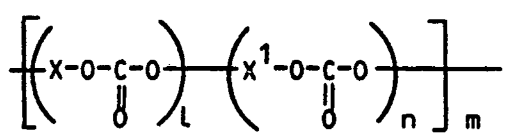

dans laquelle
$X^1$ représente des restes de polyesters aliphatiques identiques ou différents de poids moléculaire 200 à 6 000, de préférence 750 à 3 500,
X a les mêmes significations que $X^1$ ou représente des restes de polyéthers aliphatiques identiques ou différents de poids moléculaire 200 à 20 000, de préférence 700 à 10 000,
n est égal à 0 ou représente un nombre entier de 1 à 20, de préférence de 10 à 20,
l est un nombre entier de 1 à 20, de préférence de 10 à 20 et
m est un nombre entier, de préférence 20,
et la viscosité limite du polymère dans le tétrahydrofuranne est de préférence de 0,5 à 2,5 dl/g.

7. Matériau dentaire selon les revendications 2 à 6, caractérisé en ce que le composant A consiste pour 50 à 98 % en poids en esters (méth)acryliques monofonctionnels et pour 2 à 50 % en poids en esters (méth)acryliques polyfonctionnels servant d'agents réticulants.

8. Matériau dentaire selon les revendications 2 à 7, caractérisé en ce que le composant B est un polymère en perles à une dimension de particule moyenne de 5 à 150 µm.

9. Les objets moulés dentaires qu'on peut obtenir par polymérisation radicalaire des matériaux dentaires

selon les revendications 1 à 8, sous formage.

10. Procédé de préparation d'un matériau dentaire contenant en tant que liant des esters (méth)acryliques polyfonctionnels, de 5 à 50 % en poids d'une matière de charge minérale microfine et le cas échéant d'autres monomères, une matière de charge minérale et/ou organique, caractérisé en ce que l'on met en oeuvre un polyester aliphatique non réticulé ayant un poids moléculaire de $10^3$ à $5 \cdot 10^5$ et une température de transition du second ordre inférieure ou égale à 0° C et on polymérise.